# EUROPÄISCHE PATENTSCHRIFT

(11) **EP 1 503 977 B1**
(45) Veröffentlichungstag und Bekanntmachung des Hinweises auf die Patenterteilung: **05.11.2008**
(21) Anmeldenummer: 02807394.8
(22) Anmeldetag: 05.12.2002
(51) Int. Cl.: C07C 45/50, C07C 29/16

(54) **VERFAHREN ZUR RHODIUM-KATALYSIERTEN HYDROFORMYLIERUNG VON OLEFINEN UNTER REDUZIERUNG DER RHODIUMVERLUSTE**
METHOD FOR THE RHODIUM-CATALYSED HYDROFORMYLATION OF OLEFINS WITH REDUCTION OF RHODIUM LOSSES
PROCEDE D'HYDROFORMYLATION A CATALYSE AU RHODIUM D'OLEFINES AVEC REDUCTION DES PERTES DE RHODIUM

(30) Priorität: 10.05.2002 DE 10220801
(43) Veröffentlichungstag der Anmeldung: 09.02.2005
(73) Patentinhaber: Evonik Oxeno GmbH, 45772 Marl (DE)
(72) Erfinder: TÖTSCH, Walter, 45770 Marl (DE); KAIZIK, Alfred, 45772 Marl (DE); SCHULTE-ALTHOFF, Hermann-Josef, 45721 Haltern (DE)
(74) Vertreter: Hirsch, Hans-Ludwig
(86) Internationale Anmeldenummer: PCT/EP2002/013779
(87) Internationale Veröffentlichungsnummer: WO 2003/095406

(56) Entgegenhaltungen:
- US-A- 4 443 638

## Beschreibung

Die vorliegende Erfindung betrifft ein Verfahren zur Herstellung von Aldehyden und Alkoholen durch Rhodium-katalysierte Hydroformylierung von Olefinen, wobei die durch die Aufarbeitung des Austrags der Hydroformylierungsreaktion entstehenden Verluste an aktivem Rhodium-Katalysator minimiert werden.

In technischem Maßstab werden Aldehyde und/oder Alkohole durch Hydroformylierungen von Olefinen mit Kobalt- oder mit Rhodiumkatalysatoren hergestellt. Dabei ist die Verwendung von Rhodiumkatalysatoren meist vorteilhaft, da mit ihnen höhere Selektivitäten und Produktausbeuten erreicht werden können. Im Vergleich zum Kobalt ist Rhodium jedoch teurer, sodass bei der Hydroformylierung von Olefinen zu den entsprechenden Aldehyden mit Rhodium-Katalysatoren der Katalysator ein nicht unbedeutender Kostenfaktor darstellt. Zur Erhöhung der Wirtschaftlichkeit muss daher der spezifische Katalysatorverbrauch gesenkt werden. Darunter ist die Menge an Katalysator zu verstehen, die bei einem Langzeitbetrieb dem Prozeß zugeführt werden muss, um ein konstantes Aktivitätsniveau des Katalysators zu gewährleisten.

Die Rhodium-katalysierte Umsetzung von Olefinen zu den entsprechenden Aldehyden erfolgt meist in homogener, flüssiger Phase. Bei Hydroformylierungen in homogener Phase, d. h. Katalysator, Olefine, Produkte, Lösungsmittel usw. liegen in einer Phase vor, stellt sich das Problem, den Katalysator nach der Reaktion aus dem Reaktionsaustrag abzutrennen. Dies kann einfach durch Abdestillation des nicht umgesetzten Edukts und der Produkte erfolgen; der im Sumpf, meist in hochsiedenden Komponenten gelöste Katalysator, wird anschließend in den Reaktor zurückgeführt. Die Destillation kann dabei kontinuierlich oder diskontinuierlich erfolgen.

Bei der destillativen Abtrennung des Rhodium-Katalysators ist häufig eine Zersetzung oder Desaktivierung des Katalysators festzustellen. Besonders bei der Hydroformylierung längerkettiger Olefine kann die Destillation aufgrund der hohen Siedepunkte der Produkte nur noch bei erhöhter Temperatur und/oder vermindertem Druck durchgeführt werden, d. h. die erforderlichen Bedingungen zur Abtrennung des Katalysators begünstigen seine Desaktivierung.

Zur Verminderung der Katalysatordesaktivierung während der Aufarbeitung des Reaktoraustrags bei Rhodium-katalysierten Hydroformylierungsreaktionen von Olefinen sind mehrere Verfahren bekannt:
In EP 0272608 ist ein Verfahren beschrieben, bei dem ein Rhodiumkatalysator mit Triphenylphosphinoxid-Liganden eingesetzt wird. Bei der Aufarbeitung des Reaktionsaustrags wird vor dessen Destillation Triphenylphosphin (die neunfache Menge bezogen auf Rhodium) zugesetzt. Der Destillationsrückstand enthält somit Rhodiumkomplexe mit Triphenylphosphin als Liganden sowie Triphenylphoshin und Triphenylphosphinoxid. Anschließend wird das gesamte, d. h. das freie und das komplexierte Triphenylphosphin zu Triphenylphosphinoxid oxidiert und diese Katalysatorlösung in den Reaktor zurückgeführt. Für die Oxidation des Triphenylphosphins wird Sauerstoff oder ein Peroxid eingesetzt. Weitere Varianten dieser Methode sind z. B. in JP 63 222 139, JP 63 208 540, DE 3 338 340 und JP 63 218 640 beschrieben.

Diese Verfahren haben den Nachteil, das ständig Triphenylphosphin verbraucht wird. Daraus entsteht durch Oxidation die äquivalente Menge Triphenylphosphinoxid. Um dessen Konzentration in der Katalysatorflüssigkeit bzw. im Reaktor zu begrenzen, ist ein Ausschleusestrom notwendig, durch den wiederum Rhodium ausgetragen wird. Zusätzlich ist eine Oxidationsvorrichtung notwendig. Bei der Oxidation fallen, wenn nicht gerade Luft verwendet wird, Kosten für das Oxidationsmittel an.

Zur Stabilisierung der Phosphor-haltigen Liganden des Rhodiums können der Hydroformylierungsreaktion und/oder dem Aufarbeitungsschritt der Reaktionsprodukte weitere Verbindungen zugesetzt werden.

In US 5 731 472, US 5 767 321 und EP 0 149 894 werden Verfahren zur Hydroformylierung von n-Butenen beschrieben. Darin werden Rhodium-Katalysatoren eingesetzt, die PhosphitLiganden enthalten und durch Zusatz von Aminen stabilisiert werden. Nachteilig daran ist, dass Amine als Katalysatoren für Aldolkondensationen wirken können und somit die Bildung von Hochsieder begünstigt wird.

Die Hydroformylierung eines C₈-Olefingemischs, hergestellt durch Dimerisierung von Butenen, unter der Katalyse von Rhodiumkomplexen und deren Stabilisierung mit substituierten Phenolen wird in JP 04-164042 beschrieben. Dabei werden Rhodiumverbindung, Ligand und Stabilisator im molaren Verhältnis 1/10/50 eingesetzt. Nachteilig bei diesem Verfahren sind die Kosten für den Stabilisator und der Aufwand für dessen Abtrennung.

Üblicherweise wird der Rhodium-Katalysator als Lösung in einem hochsiedenden Lösungsmittel durch schonende Destillation des Austrags der Hydroformylierungsreaktion zurückgewonnen.

Bei der Hydroformylierung von Olefinen in Gegenwart von Rhodium-Katalysatoren entstehen häufig geringe Mengen an Hochsiedem als Nebenprodukte. Die Hochsieder sind Aldolisierungs- und Acetalisierungsprodukte sowie ferner - durch Disproportionierung der Aldehyde zu Säuren und Alkoholen - Ester dieser Säuren. Dieser Hochsieder verbleibt bei der Destillation des Hydroformylierungsaustrags zusammen mit den Rhodiumverbindungen im Destillationssumpf. Damit in einem kontinuierlichen Verfahren die Hochsiederkonzentration im Hydroformylierungsreaktor auf einen konstanten Wert gehalten werden kann, muss im quasistationärem Zustand gerade so viel Hochsieder abgetrennt werden, wie bei der Hydroformylierungsreaktion gebildet wird. Dies geschieht durch gezielte Ausschleusung eines Teils des Destillationssumpfes. Mit dem Ausschleusestrom werden auch Teilmengen an aktiven Rhodium-Katalysator aus dem Prozesskreislauf entfernt. Im Idealfall würde die ausgeschleuste Rhodiummenge gerade derjenigen Rhodiummenge entsprechen, die bei einem kontinuierlichen Verfahren zur Aufrechterhaltung eines stationären Zustands nachdosiert werden müsste.

Bei herkömmlichen Verfahren muss jedoch eine größere Rhodiummenge nachdosiert werden, weil während der destillativen Aufarbeitung unter anderem auf Grund der thermischen Beanspruchung unlösliche Rhodiumverbindungen, wie z. B. mehrkernige Rhodium-Cluster, und/oder metallisches Rhodium entstehen. Diese Rhodiummengen sind zwar weiterhin in der Apparatur vorhanden, sind jedoch katalytisch inaktiv und stehen nicht als Katalysator im Hydroformylierungsreaktor zur Verfügung. Darüber hinaus sind die im Sumpfprodukt der Destillation enthaltenen gelösten Rhodiumverbindungen, die in den Hydroformylierungsreaktor rückgeführt werden, weniger aktiv als ein frischer Rhodiumkatalysator, sodass selbst bei gleicher Rhodiumkonzentration im Hydroformylierungsreaktor mit rückgeführten Rhodiumverbindungen eine geringere Raum-Zeit-Ausbeute enthalten wird.

Die Wirtschaftlichkeit eines Hydroformylierungsverfahren mit Rhodium-Katalysatoren hängt also hauptsächlich vom spezifischen Rhodiumverbrauch ab. Es bestand daher die Aufgabe, ein Verfahren zur Herstellung von Aldehyden durch Rhodium-katalysierte Hydroformylierung von Olefinen zu entwickeln, das sich durch einen geringen Katalysatorverbrauch auszeichnet und ohne den Zusatz von Katalysator-stabilisierenden Substanzen durchgeführt werden kann.

Überraschender Weise wurde gefunden, dass der Katalysatorverbrauch eines Rhodium-Katalysierten Hydroformylierungsverfahrens von Olefinen stark reduziert werden kann, wenn bei der destillativen Aufarbeitung des Hydroformylierungsaustrags bestimmte Rhodiumkonzentrationen im Destillationssumpf nicht überschritten werden.

Gegenstand der vorliegenden Erfindung ist daher ein Verfahren zur Herstellung von Aldehyden und Alkoholen durch Rhodium-katalysierte Hydroformylierung von Olefinen mit 6-20 Kohlenstoffatomen mit anschließender destillativer Auftrennung des Austrags der Hydroformylierungsreaktion in die Hydroformylierungsprodukte und eine Rhodium-haltige Lösung und Rückführung dieser Lösung in die Hydroformylierungsreaktion, wobei die Rhodium-Konzentration der rückgeführten Rhodium-haltigen Lösung 20-150 Massen-ppm beträgt.

Bevorzugt beträgt die Rhodium-Konzentration der rückgeführten Rhodium-haltigen Lösung 20-100 und insbesondere 20-50 Massen-ppm Rhodium.

Zur Ausübung des erfindungsgemäßen Verfahrens sind mehrere Varianten möglich:

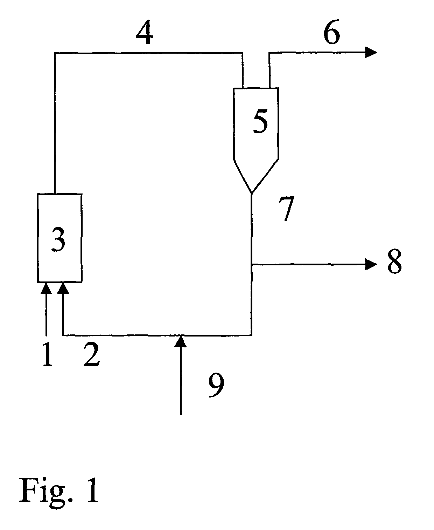

Figur 1 soll eine Variante des erfindungsgemäßen Verfahrens näher erläutern. Hier wird durch Leitung 1 Olefin und Synthesegas, sowie durch Leitung 2 Katalysatorlösung in den Hydroformylierungsreaktor 3 geleitet. Über Leitung 4 wird der Hydroformylierungsaustrag in die destillative Trennstufe 5 eingebracht, wo als Kopfprodukt 6 die gewünschten Aldehyde und Alkohole abgetrennt werden. Das Sumpfprodukt 7 enthält den Rhodiumkatalysator, und/oder Hochsieder, und/oder nicht abgetrennte Aldehyde und Alkohole und/oder inerte Lösungsmittel. Optional kann ein Teil der Rhodiumhaltigen Lösung über Leitung 8 ausgeschleust werden. Die Lösung wird in den Reaktor 3 zurückgeführt, wobei über 9 frischer Katalysator zugesetzt werden kann.

Als Lösungsmittel enthält die Rhodium-haltige Lösung die Reaktionsprodukte der Hydroformylierungsreaktion, nämlich Aldehyde, Alkohole und gebildete Hochsieder, und zugesetzte inerte Lösungsmittel ausgewählt aus Texanol, Dioctylphthalat (DOP) oder Diisononylphthalat (DINP). Die Rhodium-Konzentration wird über die destillative Auftrennung des Austrags der Hydroformylierungsreaktion, nämlich durch einen entsprechenden Anteil an Alkoholen und Aldehyden, eingestellt. Die Menge am Sumpfprodukt kann durch eine entsprechende Fahrweise der Destillationsapparatur (Temperatur, Druck) eingestellt werden. Als Lösungsmittel werden die durch die Reaktion gebildeten Hochsieder und zugesetzte inerte Lösungsmittel, jeweils alleine oder zusätzlich mit den durch die Reaktion gebildeten Alkohole und Aldehyde eingesetzt.

Die Edukte für das erfindungsgemäße Verfahren sind Olefine oder Olefingemische mit 6 bis 20 Kohlenstoffatomen sowie mit end- und/oder innenständigen C-C-Doppelbindungen. Die Gemische können aus Olefinen gleicher, ähnlicher (± 2) oder deutlich unterschiedlicher (> ± 2) C-Zahl bestehen. Als Olefine, die entweder in reiner Form, in einem Isomerengemisch oder in einem Gemisch mit weiteren Olefinen anderer C-Zahl als Edukt eingesetzt werden können, seien beispielsweise genannt: 1-, 2- oder 3-Hexen, 1-Hepten, lineare Heptene mit innenständiger Doppelbindung (2-Hepten, 3-Hepten usw.), Gemische linearer Heptene, 2- oder 3-Methyl-1-hexen, 1-Octen, lineare Octene mit innenständiger Doppelbindung, Gemische linearer Octene, 2- oder 3-Methylhepten, 1-Nonen, lineare Nonene mit innenständiger Doppelbindung, Gemische linearer Nonene, 2-, 3- oder 4-Methyloctene, 1-, 2-, 3-, 4- oder 5-Decen, 2-Ethyl-1-octen, 1-Dodecen, lineare Dodecene mit innenständiger Doppelbindung, Gemische linearer Dodecene, 1-Tetradecen, lineare Tetradecene mit innenständiger Doppelbindung, Gemische linearer Tetradecene, 1-Hexadecen, lineare Hexadecene mit innenständiger Doppelbindung, Gemische linearer Hexadecene. Geeignete Edukte sind weiterhin u. a. das bei der Dimerisierung von Propen anfallende Gemisch isomerer Hexene (Dipropen), das bei der Dimerisierung von Butenen anfallende Gemisch isomerer Octene (Dibuten), das bei der Trimerisierung von Propen anfallende Gemisch isomerer Nonene (Tripropen), das bei der Tetramerisierung von Propen oder der Trimerisierung von Butenen anfallende Gemisch isomerer Dodecene (Tetrapropen oder Tributen), das bei der Tetramerisierung von Butenen anfallende Hexadecen-Gemisch (Tetrabuten) sowie durch Cooligomerisierung von Olefinen mit unterschiedlicher C-Zahl (bevorzugt 2 bis 4) hergestellte Olefingemische, gegebenenfalls nach destillativer Trennung in Fraktionen mit gleicher oder ähnlicher (±2) C-Zahl. Weiterhin können Olefine oder Olefingemische, die durch Fischer-Tropsch-Synthese erzeugt worden sind, eingesetzt werden. Darüber hinaus können Olefine, die durch Olefin-Metathese oder durch andere technische Prozesse hergestellt worden sind, verwendet werden. Bevorzugte Edukte sind Gemische isomerer Octene-, Nonene-, Dodecene- oder Hexadecene, d. h. Oligomere von niedrigen Olefinen, wie n-Butenen, Isobuten oder Propen. Andere ebenfalls gut geeignete Edukte sind Oligomere aus C₅-Olefinen.

Im erfindungsgemäßen Verfahren können modifizierte Rhodiumkomplexe als Katalysatoren eingesetzt werden. Diese Rhodiumkatalysatoren können in Form ihrer aktiven Komplexe in den Prozess eingebracht werden, technisch ist es in der Regel aber einfacher, die aktiven Katalysatoren in situ aus stabilen, leicht lagerbaren Rhodiumverbindungen zu generieren. Geeignete Rhodiumverbindungen dafür sind zum Beispiel Rhodium(II)- und Rhodium(III)-salze, wie Rhodium(III)chlorid, Rhodium(III)nitrat, Rhodium(III)sulfat, Kalium-Rhodiumsulfat, Rhodium(II)- bzw. Rhodium(III)-carboxylat, Rhodium(II)- und Rhodium(III)acetat, Rhodium(II)octanoat, Rhodium(II)nonanoat, Rhodium(III)oxid, Salze der Rhodium(III)säure, Trisammoniumhexachlororhodat(III). Weiterhin eignen sich Rhodiumkomplexe, wie Rhodiumbiscarbonylacetylacetonat, Acetylacetonatobisethylen-rhodium(I). Besonders geeignet sind Rhodiumacetat, Rhodiumoctanoat und Rhodiumnonanoat.

Im Allgemeinen wird ungefähr 1 bis 500 und vorzugsweise 3 bis 50 Mol Ligand pro Mol Rhodium zugesetzt. Frischer Ligand kann zu jedem Zeitprodukt der Reaktion zugesetzt werden, um die Konzentration an freiem Liganden konstant zu halten.

Im erfindungsgemäßen Verfahren beträgt die Konzentration des Rhodiums im Hydroformylierungsreaktor bevorzugt 1 bis 50 Massen-ppm, insbesondere 5 bis 25 Massen-ppm.

Die Wahl der zugesetzten Liganden ist im erfindungsgemäßen Verfahren nicht beschränkt, sondern hängt vom eingesetzten Olefin und von den erwünschten Produkten ab. Bevorzugte Liganden sind Liganden die Stickstoff-, Phosphor-, Arsen- oder Antimonatome enthalten, besonders bevorzugt sind Phosphorliganden. Die Liganden können ein oder mehrzähnig sein, bei chiralen Liganden kann sowohl das Racemat als auch ein Enantiomer oder Diastereomer eingesetzt werden. Ebenfalls ist es möglich, ein Gemisch zweier oder mehrerer verschiedener Liganden zu verwenden. Als Phosphorliganden werden insbesondere diejenigen eingesetzt, die mit Rhodium weniger stabile Komplexe bilden als Triphenylphosphin, wie beispielsweise Phosphinoxide, Phosphite, Phosphonite und Phosphinite .

Beispiele für Phosphite sind Trimethylphosphit, Triethylphosphit, Tri-n-propylphosphit, Tri-i-propylphosphit, Tri-n-butylphosphit, Tri-i-butylphosphit, Tri-t-butylphosphit, Tris(2-ethylhexyl)phosphit, Triphenylphosphit, Tris(2.4-di-t-butylphenyl)phosphit, Tris(2-t-butyl-4-methoxyphenyl)phosphit, Tris(2-t-butyl-4-methylphenyl)phosphit, Tris(p-kresyl)phosphit. Außerdem sterisch gehinderte Phosphitliganden, wie sie unter anderem in EP 155 508, US 4 668 651, US 4 748 261, US 4 769 498, US 4 774 361, US 4 835 299, US 4 885 401, US 5 059 710, US 5 113 022, US 5 179 055, US 5 260 491, US 5 264 616, US 5 288 918, US 5 360 938, EP 472 071, EP 518 241 und WO 97/20795 beschriebenen werden. Bevorzugt eingesetzt werden mit jeweils 1 oder 2 Isopropyl- und/oder tert.-Butylgruppen an den Phenylringen, vorzugsweise in ortho-Position zur Phosphitestergruppierung, substituierte Triphenylphosphite.

Beispiele für Phosphonite sind Methyldiethoxyphosphin, Phenyldimethoxyphosphin, Phenyldiphenoxyphosphin, 6-Phenoxy-6H-dibenz[c,e][1,2]oxaphosphorin und dessen Derivate, in denen die Wasserstoffatome ganz oder teilweise durch Alkyl-, Arylreste oder Halogenatome ersetzt sind und Liganden die in den Patenten WO 9843935, JP 09-268152 und DE 198 10 794 und in den deutschen Patentanmeldungen DE 199 54 721 und DE 199 54 510 beschrieben werden.

Gängige Phosphinitliganden sind unter anderem in US 5 710 344, WO 95 06627, US 5 360 938, JP 07082281 beschrieben. Beispiele hierfür sind Diphenyl(phenoxy)phosphin und dessen Derivate, in denen die Wasserstoffatome ganz oder teilweise durch Alkyl-, Arylreste oder Halogenatome ersetzt sind, Diphenyl(methoxy)phosphin, Diphenyl(ethoxy)phosphin usw.

Im erfindungsgemäßen Verfahren werden die Rhodium-katalysierten Hydroformylierungen bei Drücken von 15 bis 300 bar durchgeführt, vorzugsweise bei Drücken von 15 bis 270 bar, insbesondere bei Drücken von 150 -270 bar. Der angewendete Druck hängt von der Struktur der Einsatzolefine, dem eingesetzten Rhodium-Katalysator und dem gewünschten Effekt ab. So können beispielsweise α-Olefine bei Drücken unter 64 bar mit hohen Raum-Zeit-Ausbeuten zu den entsprechenden Aldehyden umgesetzt werden. Bei Olefinen mit innenständigen Doppelbindungen, insbesondere bei verzweigten Olefinen, sind dagegen höhere Drücke zweckmäßig.

Die Temperaturen für die erfindungsgemäßen Rhodium-katalysierten Hydroformylierungen liegen im Allgemeinen im Bereich von 40°C bis 180°C, bevorzugt bei 90°C bis 160°C, insbesondere bei 130 bis 160 °C

Nach der Hydroformylierung wird der größte Teil des Synthesegases durch Druckentspannung entfernt. Aus dem flüssigem Reaktionsaustrag wird der Katalysator destillativ abgetrennt. Der Katalysator und gegebenenfalls zugesetzte Liganden, Stabilisatoren usw. verbleiben im/als Destillationsrückstand. Beim Anfahren, oder wenn im Prozess nur wenig Hochsieder gebildet wird, kann es vorteilhaft sein, ein hochsiedendes (höher siedend als Produkte und Edukte), inertes Lösungsmittel einzusetzen, in dem sich der Katalysator löst. Der im hochsiedenden Lösungsmittel gelöste Katalysator kann dann direkt in die Reaktoren zurückgefahren werden. Besonders vorteilhaft ist es, als hochsiedendes Lösungsmittel die im Prozess gebildeten hochsiedenden Nebenprodukte einzusetzen. Andere geeignete Lösungsmittel sind hochsiedene Ester, wie 2,2,4-Trimethylpentandiol-1,3-monoisobutyrat, das als Texanol im Handel ist.

Für die technische Ausführung der destillativen Katalysatorabtrennung sind verschiedene Verfahrensweisen anwendbar. Bevorzugt ist die Abtrennung der Katalysatorlösung über Fallfilm-, Kurzstrecken- oder Dünnschichtverdampfer oder Kombinationen aus diesen Apparaten. Der Vorteil einer solchen Kombination kann zum Beispiel darin liegen, in einem ersten Schritt noch gelöstes Synthesegas sowie ein Teil der Produkte und der noch vorhandenen Ausgangsolefine (zum Beispiel in einem Fallfilmverdampfer) abzutrennen, um dann in einem zweiten Schritt (zum Beispiel in einem Dünnschichtverdampfer), die endgültige Abtrennung des Katalysators vorzunehmen.

Die Destillationsdrücke liegen zwischen 5 mbar und 1 bar, vorzugsweise zwischen 10 mbar und 100 mbar. Die Destillationstemperaturen betragen 40 bis 180 °C, insbesondere 80 bis 150 °C.

Optional kann die Rhodium-haltige Lösung (Sumpfprodukt) zusätzlich mit Kohlenmonoxid stabilisiert werden, wie es in DE 100 48 301.1 beschrieben ist.

Ein Teil der Rhodium-haltigen Lösung (Sumpfprodukt) kann zur Konstanthaltung der Hochsiederkonzentration im Hydroformylierungsreaktor ausgeschleust werden. Der andere Teil des Sumpfproduktes wird in den Hydroformylierungsreaktor zurückgefahren. Mit dem Ausschleusestrom wird auch ein Teil des Katalysators ( Rhodium und Ligand) aus dem Prozess entfernt. Diese Mengen und andere Fehlmengen an Rhodium und Ligand im rückgeführten Strom müssen zur Aufrechterhaltung einer bestimmten Katalysatorkonzentration im Hydroformylierungsreaktor nachdosiert werden. Im Idealfall müssen nur diese Mengen an Rhodium-Katalysator ersetzt werden.

Optional können aus dem Ausschleusestrom weitere Produkte, beispielsweise durch Destillation, abgetrennt werden.

Aus dem Ausschleusestrom kann nach bekannten Verfahren das Rhodium zurückgewonnen werden.

Die Brüden, die bei der Aufkonzentrierung anfallen, können destillativ in die Zielprodukte, Aldehyde und Alkohole, Kohlenwasserstoffe und sonstige Nebenprodukte getrennt werden. Aus der Kohlenwasserstofffraktion können gegebenenfalls Olefine gewonnen werden, die in den Prozess zurückgeführt werden können.

Die durch das erfindungsgemäße Verfahren gewonnenen Aldehyde können als solche verwendet werden, beispielsweise als Riechstoff, zu Carbonsäuren oxidiert oder zu Alkoholen hydriert werden.

Die im erfindungsgemäßen Verfahren anfallenden Alkohole oder die durch Hydrierung der Aldehyde gewonnenen Alkohole sind Vorstufen für Ester, insbesondere Weichmacher, wie beispielsweise Phthalate, Hydrophthalate, Adipate, Citrate, Trimellitate, und für Detergenzien. Weiterhin können sie als Lösungsmittel eingesetzt werden.

Die folgenden Beispiele sollen die Erfindung erläutern, ohne ihre Anwendungsbreite einzuschränken, die sich aus der Beschreibung und den Patentansprüchen ergibt.

### Beispiel 1

### Rhodium-katalysierte Oxierung von C12-Olefin (Tributen ) mit frischem Katalysator

In einem 10 1 Autoklav wurden 5000 g Tributen aus dem Octol-Prozess bei 135 °C unter 250 bar Synthesegasdruck 4 Stunden lang in Gegenwart eines Phosphit-modifizierten Rhodiumkatalysators umgesetzt. Der aktive Rhodiumkatalysator wurde in situ aus Rhodiumoctanoat und Tris(2.4-di-tert.butylphenyl)phosphit generiert.

Die Rhodiumkonzentration (bezogen auf Gesamtreaktionmasse) wurde auf 10 ppm eingestellt, das molare Phosphor -Rhodium-Verhältnis (P/Rh) betrug 10 zu 1.

Als inertes, hochsiedendes Lösungsmittel wurden dem Reaktionsgemisch 250 g Texanol (2.2.4-Trimethylpentandiol-1.3-monoisobutyrat) zugegeben.

Der Umsatz des Olefins wurde sowohl mittels einer GC-Analyse als auch über die Menge an aufgenommenem Synthesegas verfolgt. Nach 4 Stunden wurde die Reaktion abgebrochen. Der Reaktionsaustrag enthielt 16,5 Massen-% C12-Olefin, 78,1 Massen-% C13-Aldehyd (Iso-Tridecanal), 4,3 Massen-% C13-Alkohol (Iso-Tridecanol) und 1,1 Massen-% Hochsieder. Diese Produktzusammensetztung enspricht einem Tributen-Umsatz von 81,2 % und einer Wertproduktausbeute (C13-Aldehyd/ Alkohol) von 79,7 %.

### Beispiel 2 Vergleichsbeispiel

### Rhodium-Rückgewinnung aus dem Reaktionsaustrag

Für die Rückgewinnung des Katalysators wurden 2500 g Reaktionsaustrages aus dem Beispiel 1 in einem Labor-Dünnschichtverdampfer bei 130 °C und 20 mbar aufgetrennt. Dabei wurde der Rhodium-haltige Hochsieder von den Leichtsiedern (Wertprodukte, nicht umgesetztes C13-Olefin ) abgetrennt. Unter den gewählten Trennungsbedingungen wurde im Sumpf des Dünnschichtverdampfers ein Rhodium-haltiger Hochsieder ( Sumpfaustrag ) mit einem Rhodium-Gehalt von 248 ppm erhalten.

### Beispiel 3 gemäß der Erfindung

### Rhodium-Rückgewinnung aus dem Reaktionsaustrag

Wie im Beispiel 2 wurden für die Rückgewinnung des Katalysators 2500 g Reaktionsaustrages aus dem Beispiel 1 in einem Labor-Dünnschichtverdampfer bei 130 °C und 40 mbar eingesetzt. Dabei wurde der Rhodium-haltige Hochsieder von den Leichtsiedern (Wertprodukte, nicht umgesetztes C13-Olefin) abgetrennt. Unter den im Vergleich zum Beispiel 2 gewählten, milderen Trennungsbedingungen wurde im Sumpfaustrag ein Rhodium-Gehalt von 43 ppm erzielt

### Beispiel 4 Vergleichsbeispiel

### Oxierung von C12-Olefin (Tributen ) mit rückgeführtem Katalysator

In einem 21 Autoklav wurden 1000 g Tributen aus dem Octol-Prozess bei 135 °C unter 250 bar Synthesegasdruck 4 Stunden lang in Gegenwart eines rückgeführten, gebrauchten Rhodiumkatalysators umgesetzt. Als Katalysator-Vorläufer wurde der im Beispiel 2 gewonne hochkonzentierte Rhodium-haltige Hochsieder mit 248 ppm Rhodium verwendet. Dabei wurde der Rhodium-Gehalt (bezogen auf Gesamtreaktionmasse), wie im Beispiel 1, auf 10 ppm eingestellt..Das molare Phosphor -Rhodium-Verhältnis (P/Rh) betrug 10 zu 1.

Der Umsatz des Olefins wurde sowohl mittels einer GC-Analyse als auch über die Menge an aufgenommenem Synthesegas verfolgt. Nach 4 Stunden Versuchszeit wurde ein Tributen-Umsatz von 67,3 % und eine Wertproduktausbeute (C13-Aldehyd/ Alkohol) von 66,2 %. ermittelt. Im Vergleich zum frischen Rhodium-Katalysator (Beispiel 1) ist beim Einsatz des gebrauchten Katalysators ein deutlicher Rückgang des Umsatzes und der Wertproduktausbeute festzustellen.

### Beispiel 5 Gemäß der Erfindung

### Oxierung von C12-Olefin (Tributen) mit rückgeführtem Katalysator

In einem 21 Autoklav wurden 1000 g Tributen aus dem Octol-Prozess bei 135 °C unter 250 bar Synthesegasdruck 4 Stunden lang in Gegenwart eines rückgeführten, gebrauchten Rhodiumkatalysators umgesetzt. Als Katalysator-Vorläufer wurde der im Vergleich zum Beispiel 2 wenig konzentierte Rhodium-haltige Hochsieder mit 43 ppm Rhodium aus dem Beispiel 3 verwendet. Das Rhodium-Gehalt (bezogen auf Gesamtmasse) wurde wie in den Beispielen 1 und 4, auf 10 ppm eingestellt..Das molare Phosphor -Rhodium-Verhältnis (P/Rh) betrug 10 zu 1.

Der Umsatz des Olefins wurde, wie in Beispielen 1 und 3, sowohl mittels einer GC-Analyse als auch über die Menge an aufgenommenem Synthesegas verfolgt. Nach 4 Stunden Versuchszeit wurde ein Tributen-Umsatz von 80,5 % und eine Wertproduktausbeute (C13-Aldehyd/ Alkohol) von 79,0 %. ermittelt. Im Vergleich zum frischen Katalysator (Beispiel 1) ist beim Einsatz des gebrauchten Katalysators, hergestellt aus weniger konzentierten Rhodium-haltigen Hochsieder mit 43 ppm Rh, kein nennenswerter Rückgang des Umsatzes und der Wertproduktausbeute festzustellen.

## Patentansprüche

1. Verfahren zur Herstellung von Aldehyden und Alkoholen durch Rhodium-katalysierte Hydroformylierung von Olefinen mit 6-20 Kohlenstoffatomen mit anschließender destillativer Auftrennung des Austrags der Hydroformylierungsreaktion in die Hydroformylierungsprodukte und eine Rhodium-haltige Lösung und Rückführung dieser Lösung in die Hydroformylierungsreaktion,
**dadurch gekennzeichnet,**
**dass** die Rhodium-Konzentration der rückgeführten Rhodium-haltigen Lösung 20-150 Massen-ppm beträgt, wobei die Rhodium-haltige Lösung als Lösungsmittel die durch die Hydroformylierungsreaktion gebildeten Hochsieder, Aldehyde und Alkohole und ein inertes Lösungsmittel ausgewählt aus Texanol, Dioctylphthalat oder Diisononylphthalat enthält und die Rhodium-Konzentration durch den Anteil der Aldehyde und Alkohole über die destillative Auftrennung des Austrags der Hydroformylierungsreaktion eingestellt wird.

2. Verfahren nach Anspruch 1,
**dadurch gekennzeichnet,**
**dass** die Rhodiumkatalysatoren Phosphitliganden enthalten.

3. Verfahren nach Anspruch 1 oder 2,
**dadurch gekennzeichnet,**
**dass** die Rhodiumkatalysatoren Tris(2.4-di-t-butylphenyl)phosphit als Ligand enthalten.

## Claims

1. Process for preparing aldehydes and alcohols by rhodium-catalyzed hydroformylation of olefins having 6-20 carbon atoms with subsequent separation by distillation of the output from the hydroformylation reaction into the hydroformylation products and a rhodium-containing solution and recirculation of this solution to the hydroformylation reaction,
**characterized in that**
the rhodium concentration of the recirculated rhodium-containing solution is 20-150 ppm by mass, wherein the rhodium-containing solution comprises the high boilers, aldehydes and alcohols formed in the hydroformylation reaction as solvent and an inert solvent selected from among Texanol, dioctyl phthalate and diisononyl phthalate, and the rhodium concentration is set by means of the proportion of aldehydes and alcohols via the separation by distillation of the output from the hydroformylation reaction.

2. Process according to Claim 1,
**characterized in that**
the rhodium catalysts comprise phosphite ligands.

3. Process according to Claim 1 or 2,
**characterized in that**
the rhodium catalysts comprise tris(2,4-di-t-butylphenyl) phosphite as ligand.

## Revendications

1. Procédé pour la préparation d'aldéhydes et d'alcools par une hydroformylation catalysée par du rhodium d'oléfines comprenant 6-20 atomes de carbone avec une séparation par distillation consécutive du produit évacué de la réaction d'hydroformylation en produits d'hydroformylation et en une solution contenant du rhodium et recyclage de cette solution dans la réaction d'hydroformylation, **caractérisé en ce que** la concentration en rhodium de la solution recyclée contenant du rhodium est de 20-150 ppm en masse, la solution contenant du rhodium contenant comme solvant la fraction à haut point d'ébullition, les aldéhydes et les alcools formés par la réaction d'hydroformylation et un solvant inerte, choisi parmi le texanol, le phtalate de dioctyle ou le phtalate de diisononyle et la concentration en rhodium étant réglée par la proportion des aldéhydes et des alcools via la séparation par distillation du produit évacué de la réaction d'hydroformylation.

2. Procédé selon la revendication 1, **caractérisé en ce que** les catalyseurs de rhodium contiennent des ligands de type phosphite.

3. Procédé selon la revendication 1 ou 2, **caractérisé en ce que** les catalyseurs de rhodium contiennent du tris(2,4-di-t-butylphényl)phosphite comme ligand.
